(19) Europäisches Patentamt
European Patent Office
Office européen des brevets

(11) **EP 0 775 491 A1**

## (12) EUROPEAN PATENT APPLICATION

published in accordance with Art. 158(3) EPC

(43) Date of publication:
28.05.1997 Bulletin 1997/22

(21) Application number: 96918852.3

(22) Date of filing: 19.06.1996

(51) Int. Cl.$^6$: **A61K 31/725**, A61K 35/74
// C08B37/00, C12P19/04

(86) International application number:
PCT/JP96/01696

(87) International publication number:
WO 97/00687 (09.01.1997 Gazette 1997/03)

(84) Designated Contracting States:
DE FR GB

(30) Priority: 22.06.1995 JP 180712/95

(71) Applicant: TAYCA CORPORATION
Osaka-shi Osaka-fu 551 (JP)

(72) Inventors:
• OOISO, Yoichi
Osaka 577 (JP)

• SUGIHARA, Ryosuke
Osaka 551 (JP)
• OOMORI, Hitoshi
Okayama-shi Okayama 700 (JP)

(74) Representative: Kolb, Helga, Dr. Dipl.-Chem. et al
Hoffmann, Eitle & Partner,
Patent-und Rechtsanwälte,
Arabellastrasse 4
81925 München (DE)

## (54) IMMUNOPOTENTIATOR

(57) An immunoactivating agent comprising a polysaccharide consisting of galacturonic acid, rhamnose and glucose in a molar ratio of galacturonic acid : rhamnose : glucose = 0.6 to 1.0 : 0.8 to 1.2 : 0.8 to 1.2. This immunoactivating agent has an excellent immunoactivating activity, and the polysaccharide is a microorganism-produced acidic heteropolysaccharide having higher productivity than the basidiomycete-derived polysaccharides such as krestin and lentinan.

## Description

### Field of Art

The present invention relates to an immunoactivating agent, and more particularly it relates to an immunoactivating agent comprising a microorganism-produced polysaccharide.

### Background Art

Chemical therapy is exemplified as a method for cancer treatment, but the chemical therapeutical agents used therefor usually have high toxicity and tend to produce unfavorable side effects. Under these circumstances, attention is attracted to the idea of preventing and treating cancer as well as bacterial and viral infections by enhancing immunity possessed by the organism itself, and it is desired to provide a substance having an immunoactivating function.

A variety of polysaccharides have been utilized in the fields of medicines, foods and cosmetics. Natural polysaccharides may be roughly classified into the following three types: plant-derived polysaccharides, animal-derived polysaccharides and microorganism-derived polysaccharides. Among them, polysaccharides derived from microorganisms (microorganism-produced polysaccharides) have come to be used popularly for convenience of production and supply.

$\beta$-1,3 glucans such as lentinan and krestin have been known as polysaccharides utilized for immunoactivating agent (Japanese Patent Publication (KOKOKU)) Nos. 55-12886, 56-46481, etc.). These polysaccharides are obtained as products by basidiomycetes, so that as compared with the polysaccharides produced by microorganisms, their productivity is not sufficient high, with the yield being usually less than 10% based on the cells. As an instance of acidic heteropolysaccharides having an immunoactivating activity, there is known a polysaccharic substance A29-PS disclosed in Japanese Patent Publication (KOKOKU) No. 1-59283, but its effect is not satisfactory (see the data shown in "Science and Industry", 58 (11), 407-421, 1984).

The present invention has been performed in view of the above circumstances, and its object is to provide an immunoactivating agent comprising a polysaccharide produced from a microorganism. As a result of present inventors' earnest studies, it has been found that the polysaccharides produced from the bacteria of the genus Azotobacter have excellent immunoactivating activity and can be obtained with higher productivity than the basidiomycetes-produced polysaccharides such as lentinan and krestin, and the present invention has been achieved.

### Disclosure of the Invention

Thus, the aspect of the present invention lies in an immunoactivating agent comprising a polysaccharide consisting essentially of three types of saccharides, viz. galacturonic acid, rhamnose and glucose, and having a molar ratio of galacturonic acid : rhamnose : glucose of 0.6 to 1.0 : 0.8 to 1.2 : 0.8 to 1.2.

Hereinbelow the present invention is described in detail.

In the polysaccharide used for the immunoactivating agent of the present invention, usually galacturonic acid and glucose are D-form and rhamnose is L-form. The polysaccharides used in the present invention preferably have the following characteristics besides the features mentioned above.

(1) Molecular weight

The molecular weight of the polysaccharide as measured by gel filtration chromatography is in the range of approximately $1 \times 10^3$ to $10 \times 10^6$.

(2) Bonding form

The bonding form of the respective constituting sugars is substantially 1,3-bond.

(3) Bonding configuration

The bonding configuration of D-galacturnic acid and D-glucose is $\alpha$, and the bonding configuration of L-rhamnose is $\beta$.

(4) O-acetyl group content

Part of the hydroxyl groups of the constituting sugars are substituted with O-acetyl groups, and the number of the substituted group thereof is usually not more than 1 based on one residue of the constituting sugars on the average.

The polysaccharides used in the present invention have the following properties.

(5) Form

White fibrous (freeze-dried).

(6) Solubility

Soluble in water, dilute acids, dilute alkalis and dimethyl sulfoxide (DMSO) and insoluble in methanol, ethanol and acetone.

(7) Ultra-Violet (UV) absorption spectrum

No absorption both at 280 nm and at 260 nm which are peculiar to protein (peptide) and nucleic acid, respectively are observed.

(8) IR absorption spectrum

IR absorption peaks are observed at around 3400 $cm^{-1}$, around 1620 $cm^{-1}$, around 1100 $cm^{-1}$, around 1250 $cm^{-1}$ and around 2950 $cm^{-1}$.

(9) Color reaction

Positive for phenolsulfuric acid reaction and m-phenylphenol reaction and negative for Elson-Morgan's procedure.

The molecular weight of the polysaccharide used in the present invention, the kind, compositional ratios and bonding forms of the component saccharides can be specified by liquid chromatographical analysis, chromatography, methylation or Smith's decomposition after acid hydrolysis. An example of the specifying methods is shown below.

〈Determination of molecular weight〉

Molecular weight was determined by GPC-mode high-performance liquid chromatography with a column of "Asahipak GFA-7MF" (mfd. by Asahi Chemical Industry Co., Ltd.) using a molecular weight-retention time standard curve drawn up with pullulan of the known molecular weight as standard sample and with a 0.1M $NaNO_3$ solution as mobile phase.

〈Component saccharides and their ratios〉

The above polysaccharide and the modified one in which the carboxyl group of galacturonic acid residue had been reduced were subjected to acid hydrolysis using 2M trifluoroacetic acid (TFA) at 100°C for 6 hours to prepare the corresponding alditol acetates. Each of the obtained derivatives was subjected to gas chromatographic analysis using an ECNSS-M coated column (Gaschrom Q mfd. by Wako Pure Chemical Co. Ltd.). In the case of the polysaccharide in the present invention, there are consequently detected the same compounds as the ones obtained when D-galactose, D-glucose and L-rhamnose are treated individually. By comparing the ratios of the compounds obtained when treating the said polysaccharide and the modified one in which the carboxyl group as a galacturonic acid residue is reduced, it is possible to determine the component sugars of the polysaccharide and their molar ratios.

Now, a process for preparation of the polysaccharide used for the immunoactivating agent of the present invention is described.

Usually the said polysaccharide is obtained from the microbial culture of Azotobactor beijerinkii TNM1 strain disclosed in Japanese Patent Application Laid-Open (KOKAI) No. 7-90003 (deposited under FEM BP-4194 at Life Science and Engineering Research Institute of the Agency of Industrial Science and Technology, the Ministry of International Trade and Industry) or a variant thereof.

The said variant can be generated by exposure to radiation such as ultraviolet rays or X-rays, or by a known mutagenic means, for example, introduction of a chemical mutagen such as ethylmethanesulfonic acid (EMS) or N-methyl-N'-nitro-N-nitrosoguanidine (MNNG).

An embodiment of microbial culture using the above strain is described hereinbelow.

The medium used for the microbial culture is not specifically limited as far as it is capable of growing the microorganisms of the genius Azotobacter and pertinent amounts of a carbon source, a nitrogen source, an inorganic salt and micronutrients are contained for producing the said polysaccharide.

The carbon sources usable in the present invention include glucose, lactose, maltose, xylose, mannitol, saccharose, rhamnose, arabinose, trehalose, raffinose and the like.

The nitrogen sources include synthetic compounds such as nitrates, ammonium salts and urea, and natural organic matters such as polypeptone, corn steep liquor, yeast extract, meat extract, defatted soybean extract, peptide and amino acids. The inorganic salts usable as medium ingredient include phosphates, potassium salts, sulfates and magnesium salts. As micronutrients, yeast extract, various kinds of vitamins and such can be used. If necessary, other materials such as iron salt, calcium salt, manganese salt, etc., may be added to the medium.

The state of the medium may be either solid or liquid. In case of using a liquid medium, although stationary culture may be used, it is preferable to adopt shaking culture or aerated spinner culture because of higher yield of the objective polysaccharide. The incubation pH is not specifically defined as far as it allows growth of the microorganism and production of the objective polysaccharide, but usually it is preferable to set the pH at 4 to 8. The incubation temperature is also not specified in the present invention, but normally a temperature of 20 to 35°C is preferable. As for the incubation time, a period which maximizes the production of the objective polysaccharide should be chosen, but usually a period of 1 to 7 days is preferable.

Known methods can be employed for collecting the objective polysaccharide from the culture. For instance, the microbial cells are first removed from the culture by suitable means such as centrifugation or filtration, and then an organic solvent such as methanol, ethanol, isopropanol, acetone or the like is added to the obtained culture solution to produce a precipitate. The collected precipitate is dissolved in water and then dialyzed against water, and the dialyzate is dried by suitable means such as drafting, hot-air drying, spray drying, drum drying, vacuum drying, freeze drying, etc., to recover the objective polysaccharide.

In another method, the components other than the polysaccharide are removed from the culture by ultrafiltration, and the obtained solution is subjected to the said drying operation. Further, if necessary, the obtained polysaccharide may be purified by an ordinary polysaccharide purification method to obtain a high-purity product. As purification method, there can be used various kinds of column chromatography such as ion exchange, gel filtration and affinity, precipitation or salting-out by use of a quaternary ammonium salt, and precipitation by use of an organic solvent.

The degree of polymerization of the polysaccharide used in the present invention can be changed by adjusting the preparation conditions such as culture medium composition and culture collecting method. Also, the collected or purified product may be hydrolyzed by using TFA, formic acid, hydrochloric acid or the like and adjusting the operating conditions. A preferable result can also be obtained by changing the degree of polymerization by performing heating under pressure or ultrasonic treatment. Therefore, the molecular weight of the said polysaccharide can be optionally set within the range of approximately $1 \times 10^3$ to $10 \times 10^6$. The thus obtained polysaccharide has an immunoactivating potency as shown in the Examples described later and its yield is higher than 30 to 40% on the average based on glucose or sucrose used as starting material. Further, in an oral acute toxicity test of the said polysaccharide on rats, no case of death was observed even when the rats were dosed with 5 g/kg of the polysaccharide. Also, the increase of body weight of the rats in the test group was the same as that of the control group, and absolutely no abnormality was seen in both appearance and postmortem anatomical examinations.

From the above, it is apparent that the said polysaccharide have a high safety. This fact is further supported by the comparison with $LD_{50}$ value of the commercial chemical therapeutic agents for cancer shown below.

| $LD_{50}$ (mg/kg) of commercial chemical therapeutic agents for cancer in oral administration to rats (Values given in "Therapeutical Agents: A Collection of Japan Pharmaceutical Preparations, 9th Ed. (1985), compiled by Japan Medical Information Center" (pub. by Yakuji Jiho-sha)) | |
| --- | --- |
| Cyclophosphide | 100 (male), 118 (female) |
| Cisplatin | 34.3 (male), 36.6 (female) |
| Mitomycin C | 67.4 |
| Fluorouracil | 781 |
| Carboquone | 27.3 |

### Best Mode for Carrying out the Invention

The present invention is described in more detail below by the following examples, but it should be understood that these examples are merely intended to be illustrative and not to be construed as limiting the scope of the invention.

Polysaccharide Preparation Example 1

First, 100 ml of a medium of the composition shown in Table 1 was put into a 500 ml Sakaguchi flask, and after moist heat sterilization at 121°C for 20 minutes, a platinum loopful of Azotobacter beijerinkii TNM1 strain (FERM BP-4194) which had been under liquid shaking culture in a test tube using a medium of the composition shown in Table 2 for 3 days was inoculated in the first-said medium and subjected to reciprocal shaking culture at a shaking frequency of 110 strokes per minutes at 28°C for one day.

Table 1

| Medium composition (wt%) | |
|---|---|
| Sucrose | 3% |
| Sodium nitrate | 0.3% |
| Potassium monohydrogenphosphate | 0.15% |
| Magnesium sulfate heptahydrate | 0.05% |
| Iron sulfate heptahydrate | 0.001% |
| Calcium chloride dihydrate | 0.1% |
| pH | 6.5 |

Then, 300 ml of the resulting culture was inoculated in 6 liters of a medium of the composition shown in Table 1 contained in a 10-liter jar fermentor and sterilized in the same way as described above, and was subjected to 70-hour aerated spinner culture at 28°C and an aeration rate of 6 l/min using a 5M sodium hydroxide solution while maintaining the pH of the system at 7. The spinner speed was 400 r.p.m. in the period from start till the 24th hour of incubation, and 700 r.p.m. in the period thereafter till the end (70th hour) of incubation.

The resulting culture was adjusted to pH 4.5 with 10% sulfuric acid, then moist heat sterilized at 121°C for 60 minutes and centrifuged to remove the microbial cells. The supernatant was subjected to cross-flow type ultrafiltration repeatedly until the substances other than the polysaccharide, such as residual medium components, have been removed. Ultrafiltration System "UF-LMSII" of Toso Corp. (fractional molecular weight: 100,000) was used for ultrafiltration. The concentrated solution which did not pass through the filter was freeze-dried to obtain approximately 12 g of a single polysaccharide per liter of the medium. Singleness of the polysaccharide was confirmed by GPC-mode high performance liquid chromatography.

The molecular weight of the said polysaccharide was determined by high performance liquid chromatography using a column "Asahipak GFA-7MF" (Asahi Chemical Industry Co., Ltd.) with a 0.1M sodium nitrate solution as mobile phase. From the chromatographic peak top retention time of the polysaccharide, the molecular weight of the obtained polysaccharide was approximately 500,000 by use of the molecular weight-retention time standard curve drawn up using pullulan of the known molecular weight as standard.

The said polysaccharide was hydrolyzed down to the component saccharides to prepare the corresponding aditol acetates and then subjected to a gas chromatographic analysis. The molar ratio of the component sugars as determined from the previously prepared calibration curves and the peak areas of the respective component sugars and from the galacturonic acid content measured by the m-phenylphenol method was galacturonic acid : rhamnose : glucose = 0.9 : 1 : 1.

Polysaccharide Preparation Example 2

The same cultivation procedure as in Preparation Example 1 was conducted except that the cultivation conditions were changed as follows.

That is, the medium composition shown in Table 2 was used as the medium composition for aerated spinner culture, and the incubation time was 76 hours. The spinner speed was adjusted to 250 r.p.m. till the 48th hour of incubation and 700 r.p.m. in the period thereafter till the 76th hour of incubation. At the 48th hour after start of incubation, an aqueous solution containing 0.01 wt% of iron sulfate heptahydrate and 0.05 wt% of disodium ethylenediaminetetracetate dihydrate were added aseptically to a jar fermentor.

Table 2

| Medium composition (wt%) | |
|---|---|
| Glucose | 4% |
| Sodium sulfate | 0.3% |
| Potassium monohydrogenphosphate | 0.15% |
| Magnesium sulfate heptahydrate | 0.05% |
| Iron sulfate heptahydrate | 0.001% |
| Vitamin B1 | 0.0001% |
| Nicotineamide | 0.0001% |
| Calcium pantothenate | 0.0002% |
| Biotin | 0.0000012% |
| pH | 6.5 |

The resulting culture was moist heat sterilized at 121°C for one minute, then centrifuged and further subjected to diatom earth filtration under pressure to remove the microbial cells. Thereafter the same operation as in Preparation Example 1 was conducted to obtain a single polysaccharide having a molecular weight of approximately 300,000. The yield was 13 g based on one liter of the medium.

Polysaccharide Preparation Example 3

A 1 wt% aqueous solution of the polysaccharide obtained in Preparation Example 2 was prepared, and after adjusting its pH to 12 with 10 M sodium hydroxide, the solution was stirred at room temperature for 5 hours. Thereafter, the solution was neutralized with 10 M hydrochloric acid, desalted by ultrafiltration and freeze-dried to obtain a deacetylated polysaccharide.

Preparation of polysaccharide for measuring immunoactivating potency

First, 1 wt% aqueous solution of the respective polysaccharide obtained in the above Preparation Examples were prepared. A cationic ion exchange resin was added to each solution to convert the counter ions of carboxyl group of galacturonic acid residue in the polysaccharide into hydrogen ions, and then the solution was neutralized with sodium hydroxide to replace the counter ions with sodium ions, then passed through a 0.2 $\mu$m-opening membrane filter and freeze-dried. The resulting polysaccharide preparations were subjected to the following tests.

Example 1 (Determination of cancer growth inhibitory effect)

Five ICR mice (male, 5-week-old), in which Sarcoma 180 cancer cells of $5 \times 10^6$ had been subcutaneous grafted, were prepared for each reagent to be tested. The polysaccharides obtained in Preparation Examples 1-3 were administered interperitoneally to the mice at a dose of 30 mg/kg per day continuously for 10 days, starting 24 hours after cancer cell grafting. After 5 weeks from cancer cell grafting, the weight of the solid cancer in each mouse was measured and the cancer cell growth inhibition rate was determined from the average of measurements of the five mice tested. The same test was conducted with lentinan.

The cancer cell growth inhibition percentage (%) was calculated from the following equation:

$$\text{Cancer cell growth inhibition percentage} = \frac{W_1 - W_2}{W_1} \times 100$$

$W_1$: average weight of the solid cancers in the 5 cancer cell grafted mice to which no reagent has been administered;

$W_2$: average weight of the solid cancers in case a reagent such as polysaccharide has been administered to the mice.

The results are shown in Table 3. A greater numerical value of inhibition percentage indicates a greater potency to control growth of the cancer cells.

Table 3

|  | Cancer cell growth inhibition percentage (%) |
|---|---|
| Mice to which the polysaccharide obtained in Preparation Example 1 was administered | 87 |
| Mice to which the polysaccharide obtained in Preparation Example 2 was administered | 89 |
| Mice to which the polysaccharide obtained in Preparation Example 3 was administered | 85 |
| lentinan | 100 |

A polysaccharic substance A29-PS disclosed in Japanese Patent Publication (KOKOKU) No. 1-59283, which is an acidic heteropolysaccharide like the polysaccharide in the present invention, showed a cancer cell growth inhibition percentage of approximately 30% in the same test as conducted in Example 1 described above.

From the above results, the polysaccharides in the present invention has an excellent immunoactivating potency.

Example 2 (Determination of cytotoxic T cell (CTL) inducibility)

The following test was conducted on the C3H mice and BALB/c mice which are different in H-2 haplotype.

The spleen lymphocytes ($2 \times 10^7$) of the BALB/c mice were immunized in the abdominal cavities of the C3H mice (4 mice per group: N = 4). Each of the test specimens shown in Table 4 was given subcutaneously to the back of each of the said C3H mice at a dose of 50 mg/kg or 20 mg/kg, once a day, four times in all, from the day before immunization till 2 days after immunization. As control, physiological saline was used instead of the test specimens. After 9 days, the spleen lymphocyte suspension was collected from the C3H mice. This lymphocyte suspension contained the cytotoxic T cells (hereinafter referred to as effector cells and abbreviated as E). Then, $10^4$ cells of myeloma cell strain NS-1/Z (hereinafter abbreviated as T) derived from the BALB/c mouse into which $\beta$-galactosidase ($\beta$-gal) of Escherichia coli had been introduced, were mixed as target cells with the effector cells so that the E/T ratio would become 60 or 120, and the mixture was kept in 0.2 ml of a medium RPMI-1640 at 37°C for 4 hours. The activity of $\beta$-gal released into the supernatant by destruction of the target cells was measured, and the cytotoxicity by CTL was calculated from the following equation. The immunoactivating activity is shown in Table 4.

$$\text{Cytotoxicity (\%)} = \frac{A - B}{C - B} \times 100$$

A: released $\beta$-gal activity
B: naturally released $\beta$-gal activity
(enzyme release observed when no effector cells are present)
C: overall $\beta$-gal activity
(T used is dissolved in 0.0425% Triton-X100 and the overall enzyme activity is measured.)

$$\text{Immunoactivating activity} = \frac{\text{cytotoxity (\%) in case where a test specimen was administrated}}{\text{cytotoxity (\%) in case where a physiological saline was administrated}}$$

Table 4

| | Dosage | Immunoactivating activity | |
|---|---|---|---|
| Specimen | (mg/kg/day) | E/T=60 | E/T=120 |
| Polysaccharide obtained in Preparation Example 1 | 50 | 1.8 | 2.1 |
| Polysaccharide obtained in Preparation Example 2 | 50 | 1.4 | 1.4 |
| Polysaccharide obtained in Preparation Example 3 | 50 | 1.9 | 1.9 |
| Schizophyllan | 20 | 2.0 | 1.7 |

Example 3 (Measurement of tumor necrosis factor (TNF) interleukin (IL-6) inducibility)

First, 100 μg/0.2 ml of MDP (N-acetylmuramyl-L-alanyl-D-isoglutamine) was injected intravenously to the C3H/HeN mice (6- to 12-week-old) in groups of three, and 4 hours later, the polysaccharide obtained in Preparation Example 2 and schizophyllan, which is a commercial polysaccharide having immunoactivating potency (produced by Taito Co., Ltd. and sold by Kaken Pharmaceutical Co., Ltd.), were injected intravenously at a dose of 100 μg/0.2 ml. These mice were left under observation for anaphylaxis-like reaction and sudden death. After 90 minutes from the injection, blood was collected from the surviving mice to determine TNF and IL-6 activities in the serum. TNF activity was calculated on the basis of the ratio of activity of the actinomycin D-treated L-929 cells to that of rHuTNF (human recombinant TNF) used as control, with the killing action of the said L-929 cells being taken as index, and indicated by ng/ml. In the case of the IL-6 activity, the IL-6 dependent MH60.BSF2 cell growth stimulating action was taken as index, and the IL-6 activity was calculated on the basis of the calibration curves obtained from the test of IL-6 and control rHuIL-6 and indicated by U/ml. The results represented by average value ± standard deviation are shown in Table 5.

Table 5

| | Anaphylaxis-like reaction | Sudden death | TNF(ng/ml) | IL-6 (U/ml) |
|---|---|---|---|---|
| Polysaccharide obtained in Preparation Example 2 | 0/3 | 0/3 | 71.8 | 6,800 |
| Schizophyllan | 0/3 | 0/3 | 0 | 14.1 |

As seen from the above table, the polysaccharides in the present invention have a TNF and IL-6 inducibility which is not possessed by schizophyllan.

As described above, according to the present invention, there can be provided an immunoactivating agent having a prominent effect with higher productivity than the polysaccharides derived from basidiomycete such as krestin and lentinan. According to the present invention, there is also provided an immunoactivating agent whose toxicity is very low as compared with the conventional chemical therapeutical agents for cancer and which has the functions not observed with schizophyllan which is known to have an immunoactivating potency. Therefore, the immunoactivating agent of the present invention is expected to be applicable to prevention and treatment of cancer, prevention and treatment of microbial or viral infections, recovery of postoperative reduction of immunity and like uses.

**Claims**

1. An immunoactivating agent comprising a polysaccharide consisting essentially of galacturonic acid, rhamnose and glucose in a molar ratio of galacturonic acid : rhamnose : glucose = 0.6 to 1.0 : 0.8 to 1.2 : 0.8 to 1.2.

2. An immunoactivating agent according to Claim 1, wherein the molecular weight of the polysaccharide measured by gel filtration chromatography is approximately $1 \times 10^3$ to $10 \times 10^6$.

3. An immunoactivating agent according to Claim 1, wherein the bonding form of the constituting sugars is substantially 1,3-bond.

4. An immunoactivating agent according to Claim 1, wherein the bonding configuration of D-galacturonic acid and D-glucose is α, and the bonding configuration of L-rhamnose is β.

## INTERNATIONAL SEARCH REPORT

| | International application No. |
|---|---|
| | PCT/JP96/01696 |

**A. CLASSIFICATION OF SUBJECT MATTER**

Int. Cl$^6$ A61K31/725, A61K35/74 // C08B37/00, C12P19/04

According to International Patent Classification (IPC) or to both national classification and IPC

**B. FIELDS SEARCHED**

Minimum documentation searched (classification system followed by classification symbols)

Int. Cl$^6$ A61K31/725, A61K35/74

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP, 61-225125, A (Nakano Suten K.K.), October 6, 1986 (06. 10. 86)(Family: none) | 1 - 6 |
| A | JP, 60-181020, A (Snow Brand Milk Products Co., Ltd.), September 14, 1985 (14. 09. 85)(Family: none) | 1 - 6 |
| A | JP, 60-62995, A (Snow Brand Milk Products Co., Ltd.), April 11, 1985 (11. 04. 85)(Family: none) | 1 - 6 |
| A | JP, 60-23319, A (Nakano Suten K.K.), February 5, 1985 (05. 02. 85)(Family: none) | 1 - 6 |
| A | JP, 58-203913, A (Meiji Milk Products Co., Ltd.), November 28, 1983 (28. 11. 83)(Family: none) | 1 - 6 |
| A | JP, 58-129001, A (Takeda Chemical Industries, Ltd.), August 1, 1983 (01. 08. 83)(Family: none) | 1 - 6 |

[X] Further documents are listed in the continuation of Box C.    [ ] See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| August 28, 1996 (28. 08. 96) | September 10, 1996 (10. 09. 96) |

| Name and mailing address of the ISA/ | Authorized officer |
|---|---|
| Japanese Patent Office | |
| Facsimile No. | Telephone No. |

Form PCT/ISA/210 (second sheet) (July 1992)

<div style="text-align:center">

**INTERNATIONAL SEARCH REPORT**

</div>

| | International application No. |
|---|---|
| | PCT/JP96/01696 |

| C (Continuation).   DOCUMENTS CONSIDERED TO BE RELEVANT | | |
|---|---|---|
| Category* | Citation of document, with indication, where appropriate, of the relevant passages | Relevant to claim No. |
| A | JP, 53-66442, A (Takuma Sasaki and another), June 13, 1978 (13. 06. 78) & DE, 2751570, A & FR, 2421617, A & GB, 1595596, A & US, 4454315, A | 1 - 6 |

Form PCT/ISA/210 (continuation of second sheet) (July 1992)